# EUROPEAN PATENT APPLICATION

(11) **EP 4 631 619 A1**
(43) Date of publication of application: **15.10.2025**
(21) Application number: 23900685.1
(22) Date of filing: 06.12.2023
(51) Int. Cl.: B01J 23/44, B01J 35/58, B01J 37/02, B01J 37/08

(54) **METHOD FOR PRODUCING SUPPORTED PALLADIUM CATALYST, AND SUPPORTED PALLADIUM CATALYST**

(30) Priority: 06.12.2022 WO PCT/JP2022/044967; 23.03.2023 WO PCT/JP2023/011549
(71) Applicant: Sajiki, Hironao, Gifu-shi, Gifu 501-1196 (JP); Ikawa, Takashi, Gifu-shi, Gifu 501-1196 (JP); Yamada, Tsuyoshi, Gifu-shi, Gifu 501-1196 (JP); NGK Insulators, Ltd., Nagoya-shi, Aichi 467-8530 (JP)
(72) Inventor: YOKOYAMA Takashi, Nagoya-shi, Aichi 467-8530 (JP); NIWA Kosuke, Nagoya-shi, Aichi 467-8530 (JP); SAJIKI Hironao, Gifu-shi, Gifu 501-1196 (JP); IKAWA Takashi, Gifu-shi, Gifu 501-1196 (JP); YAMADA Tsuyoshi, Gifu-shi, Gifu 501-1196 (JP)
(74) Representative: Mewburn Ellis LLP
(86) International application number: PCT/JP2023/043629
(87) International publication number: WO 2024/122573

(57) **Abstract**

Provided are a method of producing a supported palladium catalyst that can cause palladium to be stably supported on a carrier, and a supported palladium catalyst. The method of producing a supported palladium catalyst according to an embodiment of the present disclosure includes the steps of: oxidizing a palladium compound by heating; dissolving the palladium compound after the heating in a solvent to prepare a palladium compound solution; and bringing the palladium compound solution into contact with a carrier.

## Description

### Technical Field

The present disclosure relates to a method of producing a supported palladium catalyst and a supported palladium catalyst.

### Background Art

The production of various industrial products such as a pharmaceutical by organic synthesis has been known. In such organic synthesis, it has been investigated that a supported palladium catalyst obtained by causing palladium to be supported on a carrier is used (see, for example, Patent Literature 1).

### Citation List

### Patent Literature

[PTL 1] JP 2012-125675 A

### Summary of Invention

### Technical Problem

In recent years, the applications of a supported palladium catalyst have been diversified, and the adoption of a carrier in the supported palladium catalyst in accordance with the application has been desired. However, it may become difficult to cause palladium to be sufficiently supported on the carrier depending on the kind of the carrier.

A primary object of the present disclosure is to provide a method of producing a supported palladium catalyst that can cause palladium to be stably supported on a carrier, and a supported palladium catalyst.

### Solution to Problem

[1] A method of producing a supported palladium catalyst according to an embodiment of the present disclosure includes the steps of: oxidizing a palladium compound by heating; dissolving the palladium compound after the heating in a solvent to prepare a palladium compound solution; and bringing the palladium compound solution into contact with a carrier.
[2] In the method of producing a supported palladium catalyst according to the above-mentioned item [1], the palladium compound may contain a palladium(0) complex containing zerovalent palladium.
[3] In the method of producing a supported palladium catalyst according to the above-mentioned item [2], the palladium(0) complex may contain tris(dibenzylideneacetone)dipalladium and/or tetrakis(triphenylphosphine)palladium.
[4] A supported palladium catalyst according to another aspect of the present disclosure includes: a glass fiber serving as a carrier; and palladium supported on a surface of the glass fiber.

### Advantageous Effects of Invention

According to the embodiments of the present disclosure, the method of producing a supported palladium catalyst that can cause palladium to be stably supported on a carrier, and the supported palladium catalyst can be achieved.

### Brief Description of Drawings

FIG. 1 is a backscattered electron image (BED) photograph of a supported palladium catalyst of Example 1.
FIG. 2 is a BED photograph of a supported palladium catalyst of Example 2.
FIG. 3 is a BED photograph of a supported palladium catalyst of Example 3.
FIG. 4 is a BED photograph of a supported palladium catalyst of Example 4.

### Description of Embodiments

### A. Method of producing Supported Palladium Catalyst

A method of producing a supported palladium catalyst according to one embodiment of the present disclosure includes the steps of: oxidizing a palladium compound by heating (heating step); dissolving the palladium compound after the heating in a solvent to prepare a palladium compound solution (solution preparation step); and bringing the palladium compound solution into contact with a carrier (supporting step).

According to such method, the palladium compound is oxidized by heating before being dissolved in a solvent, and hence palladium can be stably supported on the carrier. Accordingly, the degree of freedom of the carrier applicable to the supported palladium catalyst can be improved, and supporting reproducibility can be improved. As a result, a supported palladium catalyst including a carrier appropriate for its application, the supported palladium catalyst exhibiting an excellent reduction property, can be smoothly produced.

### A-1. Heating Step

In the heating step, the palladium compound is typically heated under an oxidizing atmosphere (typically air).

Examples of the palladium compound include a palladium(IV) compound containing tetravalent palladium, a palladium(II) compound containing divalent palladium, and a palladium(0) complex containing zerovalent palladium.

Examples of the palladium(IV) compound include hexachloropalladic acid, ammonium hexachloropalladate, sodium hexachloropalladate, and potassium hexachloropalladate.

Examples of the palladium(II) compound include palladium oxide, palladium sulfide, palladium chloride, palladium bromide, palladium iodide, palladium hydroxide, palladium nitrate, palladium sulfate, palladium cyanide, palladium acetate, bis(acetylacetonato)palladium, diamminedichloropalladium, diamminedinitropalladium, tetraamminepalladium chloride, tetraamminepalladium bromide, tetraamminepalladium nitrate, tetrachloropalladic acid, ammonium tetrachloropalladate, sodium tetrachloropalladate, potassium tetrachloropalladate, lithium tetrachloropalladate, potassium tetracyanopalladate, potassium tetrathiocyanatopalladate, bis(acetonitrile)dichloropalladium, bis(triphenylphosphine)palladium dichloride, and palladium trifluoroacetate.

Examples of the palladium(0) complex include tris(dibenzylideneacetone)dipalladium and tetrakis(triphenylphosphine)palladium.

Those palladium compounds may be used alone or in combination thereof.

Of the palladium compounds, for example, a palladium(II) compound and a palladium(0) complex are preferred, bis(triphenylphosphine)palladium dichloride, palladium nitrate, palladium acetate, tris(dibenzylideneacetone)dipalladium, and tetrakis (triphenylphosphine)palladium are more preferred, and tris(dibenzylideneacetone)dipalladium and/or tetrakis(triphenylphosphine)palladium is still more preferred. When the palladium compound contains a palladium(II) compound and/or a palladium(0) complex, a supported palladium catalyst can be stably produced.

The heating conditions of the palladium compound may be adjusted to any appropriate conditions in accordance with the palladium compound to be heated.

The heating pressure of the palladium compound is typically normal pressure (0.1 MPa).

The heating temperature of the palladium compound is, for example, from 50°C to 100°C, preferably from 70°C to 90°C.

The heating time of the palladium compound is, for example, from 1 hour to 24 hours, preferably from 10 hours to 20 hours.

When the heating temperature and/or heating time of the palladium compound falls within such ranges, the supporting reproducibility can be stably improved.

### A-2. Solution Preparation Step

In the solution preparation step, the palladium compound after the heating (oxidized palladium compound) is dissolved in a solvent to prepare a palladium compound solution.

Any appropriate solvent capable of dissolving the palladium compound after the heating (oxidized palladium compound) may be adopted as the solvent. The solvent is typically an organic solvent. Examples of the organic solvent include: halogenated aliphatic hydrocarbons, such as chloroform, dichloromethane, and carbon tetrachloride; alcohols, such as methanol, ethanol, and isopropanol; esters, such as methyl acetate, ethyl acetate, and butyl acetate; ethers, such as diethyl ether and dioxane; nitriles such as acetonitrile; and polar aprotic solvents, such as dimethyl sulfoxide and dimethylacetamide.

Those solvents may be used alone or in combination thereof. Of the solvents, for example, halogenated aliphatic hydrocarbons, alcohols, esters, and polar aprotic solvents are preferred.

A palladium concentration in the palladium compound solution is, for example, 0.01 mass% or more, preferably 0.15 mass% or more, more preferably 0.80 mass% or more. Meanwhile, the palladium concentration in the palladium compound solution is, for example, 2.0 mass% or less, preferably 0.5 mass% or less.

### A-3. Supporting Step

Next, the above-mentioned palladium compound solution is typically brought into contact with a carrier under an inert gas (e.g., nitrogen or argon) atmosphere. Thus, palladium is supported on a surface of the carrier.

The carrier includes any appropriate inorganic material in accordance with the application of the supported palladium catalyst. A typical example of the inorganic material is a ceramics material (non-metal and inorganic solid materials).

Examples of the ceramics material include: glass materials, such as silica glass (quartz glass), soda lime silica glass, and borosilicate glass; non-metal materials, such as silicon and carbon; and inorganic compound materials, such as aluminum oxide, silicon oxide, magnesium oxide, silicon carbide (SiC), a Si-SiC composite material, aluminum nitride, and titanium oxide.

In one embodiment, the carrier contains aluminum oxide, silicon oxide, and magnesium oxide. A content ratio of aluminum oxide in the carrier is from 15 mass% to 45 mass%. A content ratio of silicon oxide in the carrier is from 40 mass% to 60 mass%. A content ratio of magnesium oxide in the carrier is from 5 mass% to 30 mass%. When the carrier having the above-mentioned specific composition is used for the production of the supported palladium catalyst, a supported palladium catalyst having an excellent selective reduction property and being applicable to flow synthesis can be produced.

In this embodiment, the carrier is typically substantially free of calcium oxide. The phrase "substantially free" as used herein encompasses not only a case in which the carrier is completely free of calcium oxide but also a case in which the content ratio of calcium oxide in the carrier is 1 mass% or less. The content ratio of calcium oxide in the carrier is preferably 0 mass%.

The carrier more preferably contains only aluminum oxide, silicon oxide, and magnesium oxide as oxides. The carrier particularly preferably includes cordierite (2MgO·2Al₂O₃·5SiO₂).

The carrier has any appropriate form in accordance with the application of the supported palladium catalyst. Examples of the form of the carrier include a fibrous form, a particulate form, a plate form, and a thin-film form.

Those carriers may be used alone or in combination thereof.

In one embodiment, the carrier has a fibrous form.

Examples of the carrier having a fibrous form (fibrous carrier) include a carbon fiber and a glass fiber. Of those, a glass fiber is preferred. An outer diameter of the fibrous carrier is, for example, from 1 µm to 30 µm.

When the carrier has a fibrous form, the fibrous carrier is typically immersed in the above-mentioned palladium compound solution so that the palladium compound solution is brought into contact with a surface of the fibrous carrier. Thus, palladium is supported on the fibrous carrier.

An immersion temperature is, for example, 10°C or more and 40°C or less. An immersion time is, for example, 0.5 hour or more, preferably 12 hours or more. Meanwhile, the immersion time is, for example, 120 hours or less, preferably 48 hours or less.

In another embodiment, the carrier has a particulate form. The carrier having a particulate form is typically prepared by subjecting the above-mentioned ceramics material to calcination and pulverization by any appropriate method.

Examples of the carrier having a particulate form (particulate carrier) include silicon powder, silicon carbide powder, Si-SiC composite material powder, and carbon particles.

The average primary particle diameter of the particulate carrier is, for example, from 60 µm to 500 µm, and is, for example, from 60 µm to 110 µm.

When the carrier has a particulate form, the particulate carrier is typically added to the above-mentioned palladium compound solution and stirred so that the palladium compound solution is brought into contact with a surface of the particulate carrier. Thus, a dispersion liquid in which palladium is supported on the particulate carrier, and in which the particulate carrier for supporting the palladium is dispersed in the solvent is prepared.

A stirring temperature is, for example, 10°C or more and 40°C or less. A stirring time is, for example, 0.5 hour or more, preferably 12 hours or more. Meanwhile, the stirring time is, for example, 120 hours or less, preferably 48 hours or less.

The addition amount of the carrier in the supporting step is, for example, 5 parts by mass or more, preferably 20 parts by mass or more, and is, for example, 80 parts by mass or less, preferably 50 parts by mass or less with respect to 1 part by mass of palladium atoms in the palladium compound.

The addition amount of the carrier is, for example, 300 parts by mass or more, preferably 3,000 parts by mass or more, and is, for example, 50,000 parts by mass or less, preferably 10,000 parts by mass or less with respect to 100 parts by mass of the palladium compound solution.

### A-4. Solvent Removal Step

In one embodiment, the method of producing a supported palladium catalyst further includes a solvent removal step. In the solvent removal step, the solvent is removed from the carrier for supporting the palladium.

When the carrier has a fibrous form, the solvent is typically removed from the fibrous carrier by any appropriate method after the fibrous carrier for supporting the palladium is pulled up from the palladium compound solution. An example of the method of removing the solvent is evaporation under reduced pressure. After that, washing is performed as required, followed by drying.

When the carrier has a particulate form, the solvent is typically removed from the above-mentioned dispersion liquid by any appropriate method. Examples of the method of removing the solvent include filtration and evaporation under reduced pressure. Of those, filtration is preferred. When a filtrate and a residue (solid content) are separated from each other by filtration, an impurity and the like dissolved in the solvent can be collectively separated from the residue (solid content). Thus, the solid content in the dispersion liquid is recovered. After that, washing is performed as required, followed by drying.

Thus, a supported palladium catalyst is produced.

### B. Supported Palladium Catalyst

A supported palladium catalyst includes palladium serving as an active component and a carrier for supporting the palladium.

In one embodiment, the supported palladium catalyst includes palladium and a fibrous carrier (preferably a glass fiber) for supporting the palladium. In addition, in another embodiment, the supported palladium catalyst includes palladium and a particulate carrier for supporting the palladium.

The palladium in the supported palladium catalyst may have any appropriate configuration. The palladium supported on the carrier may be in a metal state, or may be in a state of being incorporated in a compound.

The valence of the palladium is, for example, 0 or more and 6 or less, preferably 0 or more and 2 or less, more preferably 0. When the valence of the palladium in the palladium compound is 2 or more, reducing treatment may be performed with any appropriate reducing agent (e.g., a hydrazine aqueous solution). Thus, the palladium having a valence of 2 or more can be reduced to palladium (0) .

When the valence of the palladium falls within the above-mentioned ranges, the activity of the supported palladium catalyst can be improved.

The amount of the palladium supported in the supported palladium catalyst is, for example, 0.5 mass% or more, preferably 1.0 mass% or more. Meanwhile, the upper limit of the amount of the palladium supported in the supported palladium catalyst is typically 10.0 mass%. The amount of the palladium supported may be measured by a mass change of a palladium source before and after the supporting. When the amount of the palladium supported falls within the above-mentioned ranges, an excellent reduction property can be stably exhibited in the supported palladium catalyst.

The palladium may be dispersed and supported in a particulate form on a surface of the carrier. The palladium supported in a particulate form on the carrier may contain flat particles. In addition, the palladium supported in a particulate form may also contain fine particles smaller than the flat particles in addition to the flat particles.

With such configuration, an excellent reduction property (selective reduction property) can be exhibited in the supported palladium catalyst.

### B-1. Reduction Property of Supported Palladium Catalyst

In one embodiment, the supported palladium catalyst is a heterogeneous catalyst, and is capable of reducing a specific functional group (typically a nitro group).

### B-2. Functional Group that can be selectively reduced (Reduction Target Functional Group)

In addition, the supported palladium catalyst may have a selective reduction property depending on the kind of the carrier. For example, when the carrier contains aluminum oxide, silicon oxide, and magnesium oxide at the above-mentioned ratio, the supported palladium catalyst has a selective reduction property.

In one embodiment, the supported palladium catalyst is capable of selective reduction of at least one reduction target functional group, and causes selective non-reduction of at least one reduction non-target functional group. The term "selective reduction" as used herein means that a conversion rate in a reduction reaction is, for example, 85% or more, or, for example, 90% or more. The term "selective non-reduction" as used herein means that the conversion rate in the reduction reaction is, for example, 15% or less, or, for example, 10% or less.

Examples of the reduction target functional group that can be reduced by the supported palladium catalyst include an alkynylene group, an alkenylene group, an alkynyl group, an alkenyl group, an azide group, a nitro group, a carbobenzoxy group serving as a protective group for an amino group, an aromatic aldehyde group, a trialkylsilyloxy group, an arylalkyloxy group bonded to carbonyl carbon, an alkylene group bonded to aromatic carbon, an arylalkyloxy group bonded to aromatic carbon, a halogeno group bonded to aromatic carbon, and an aromatic carbonyl group. Examples of such reduction target functional group include "functional groups to be selectively reduced" described in JP 2015-180494 A, the description of which is incorporated herein by reference.

Examples of the alkynylene group include alkynylene groups described in paragraph [0022] of JP 2015-180494 A. Of those, an ethynylene group, a propynylene group, a butynylene group, a pentynylene group, and a hexynylene group are preferred, and an ethynylene group and a propynylene group are more preferred.

Examples of the alkenylene group include alkenylene groups described in paragraph [0023] of JP 2015-180494 A. Of those, a vinylene group (ethenylene group), a propenylene group, a butenylene group, and a pentenylene group are preferred, and an ethenylene group and a vinylene group (ethenylene group) are more preferred.

Examples of the alkynyl group include alkynyl groups described in paragraph [0024] of JP 2015-180494 A. Of those, an ethynyl group, a propynyl group, a butynyl group, a pentynyl group, a hexynyl group, a heptynyl group, and an octynyl group are preferred, and an ethynyl group, a propynyl group, a butynyl group, a pentynyl group, and a hexynyl group are more preferred.

Examples of the alkenyl group include alkenyl groups described in paragraph [0025] of JP 2015-180494 A. Of those, a vinyl group (ethenyl group), a propenyl group (allyl group), a butenyl group, a pentenyl group, a hexenyl group, a heptenyl group, an octenyl group, a methylvinyl group, a methylpropenyl group, a methylbutenyl group, a methylpentenyl group, a methylhexenyl group, a methylheptenyl group, and a methyloctenyl group are preferred, and a vinyl group (ethenyl group), a propenyl group (allyl group), a methylvinyl group (methylethenyl group), a hexenyl group, an octenyl group, and a methylheptenyl group are more preferred.

The carbobenzoxy group serving as a protective group for an amino group is a carbobenzoxy group (benzyloxycarbonyl group) bonded to a nitrogen atom, and has a structure represented by the general formula [20] of JP 2015-180494 A.

The aromatic aldehyde group is an aldehyde group bonded to an aromatic ring. Examples of the aromatic ring include aromatic rings described in paragraph [0028] of JP 2015-180494 A. Of those, a phenyl group and a naphthyl group are preferred, and a phenyl group is more preferred.

Examples of the trialkylsilyloxy group include trialkylsilyloxy groups described in paragraph [0029] of JP 2015-180494 A. Of those, a triethylsilyloxy group, a tri-n-propylsilyloxy group, and a tri-n-butylsilyloxy group are preferred, and a triethylsilyloxy group and a tri-n-propylsilyloxy group are more preferred.

The arylalkyloxy group bonded to carbonyl carbon is directly bonded to a carbon atom in a carbonyl group (carbonyl carbon), and includes: an aryl group; an alkylene group bonded to an aryl group; and an oxygen atom for linking an alkylene group and carbonyl carbon.

As the aryl group, for example, a phenyl group and a naphthyl group are preferred, and a phenyl group is more preferred.

Examples of the alkylene group include alkylene groups each having 1 to 8 carbon atoms. Of those, alkylene groups each having 1 to 4 carbon atoms are more preferred, and a methylene group is still more preferred.

The arylalkyloxy group is particularly preferably a benzyloxy group.

The oxyalkylene group bonded to aromatic carbon is an oxyalkylene group bonded to the above-mentioned aromatic ring. Examples of the oxyalkylene group include an oxyethylene group and an oxypropylene group. Of those, an oxyethylene group is preferred. The aromatic ring is preferably a phenyl group.

The arylalkyloxy group bonded to aromatic carbon is an arylalkyloxy group bonded to the above-mentioned aromatic ring, and is preferably, for example, a benzyloxy group. The aromatic ring is preferably, for example, a phenyl group.

Examples of the halogeno group bonded to aromatic carbon is a halogen atom bonded to the above-mentioned aromatic ring. Examples of the halogen atom include fluorine, chlorine, bromine, and iodine. Of those, chlorine is preferred. The aromatic ring is preferably, for example, a phenyl group.

The aromatic carbonyl group is a carbonyl group bonded to the above-mentioned aromatic ring. The aromatic ring is preferably, for example, a phenyl group.

B-3. Functional Group subjected to Selective Non-reduction (Reduction Non-target Functional Group)

Examples of the reduction non-target functional group include an alkyloxy group (alkoxy group), an alkyloxycarbonyl group (alkoxycarbonyl group), an alkylcarbonyl group, a halogeno group, a hydroxy group, a hydroxyalkyl group, an arylalkyloxy group bonded to a carbon atom in an aromatic ring (aromatic carbon), an arylalkyloxy group bonded to a carbon atom in an aliphatic hydrocarbon group (aliphatic carbon), a tertbutyldimethylsilyloxy group, and a triisopropylsilyloxy group.

Examples of the alkyloxy group (alkoxy group) include alkoxy groups described in paragraph [0032] of JP 2015-180494 A. Of those, a methoxy group, an ethoxy group, a n-propoxy group, and an isopropoxy group are preferred, and a methoxy group is more preferred.

Examples of the alkyloxycarbonyl group (alkoxycarbonyl group) include alkoxycarbonyl groups described in paragraph [0033] of JP 2015-180494 A. Of those, a methoxycarbonyl group, an ethoxycarbonyl group, a n-propoxycarbonyl group, and an isopropoxycarbonyl group are preferred, and a methoxycarbonyl group and an ethoxycarbonyl group are more preferred.

Examples of the alkylcarbonyl group include alkylcarbonyl groups described in paragraph [0034] of JP 2015-180494 A. Of those, a methylcarbonyl group, an ethylcarbonyl group, a n-propylcarbonyl group, an isopropylcarbonyl group, a n-butylcarbonyl group, an isobutylcarbonyl group, a secbutylcarbonyl group, a tert-butylcarbonyl group, a n-pentylcarbonyl group, an isopentylcarbonyl group, a secpentylcarbonyl group, a tert-pentylcarbonyl group, a neopentylcarbonyl group, a n-hexylcarbonyl group, an isohexylcarbonyl group, a sec-hexylcarbonyl group, and a terthexylcarbonyl group are preferred, a methylcarbonyl group, an ethylcarbonyl group, a n-propylcarbonyl group, and an isopropylcarbonyl group are more preferred, and a methylcarbonyl group is still more preferred.

The halogeno group serving as the reduction non-target functional group is typically bonded to aliphatic carbon. Examples of the halogeno group include fluorine, chlorine, bromine, and iodine. Of those, chlorine and iodine are preferred.

Examples of the hydroxyalkyl group include hydroxyalkyl groups described in paragraph [0035] of JP 2015-180494 A. Of those, a hydroxymethyl group, a hydroxyethyl group, a hydroxyisopropyl group, and a hydroxy-n-propyl group are preferred, and a hydroxymethyl group is more preferred.

The arylalkyloxy group serving as the reduction non-target functional group is typically bonded to aliphatic carbon. Examples of the arylalkyloxy group bonded to aromatic carbon or aliphatic carbon include the above-mentioned arylalkyloxy groups. Of those, a benzyloxy group is preferred.

In one embodiment, the supported palladium catalyst is capable of selective reduction of all of the above-mentioned reduction target functional groups, and causes selective non-reduction of the above-mentioned reduction non-target functional groups.

In addition, as long as the supported palladium catalyst is capable of selective reduction of at least one functional group out of the above-mentioned reduction target functional groups, the other functional groups may be reduction non-target functional groups subjected to selective non-reduction.

In one embodiment, the supported palladium catalyst is capable of reducing an alkynylene group, an alkenylene group, an alkynyl group, an alkenyl group, and an azide group out of the above-mentioned reduction target functional groups, and causes selective non-reduction of a nitro group, a carbobenzoxy group serving as a protective group for an amino group, an aromatic aldehyde group, and an arylalkyloxy group bonded to carbonyl carbon in addition to the above-mentioned reduction non-target functional groups.

In another embodiment, the supported palladium catalyst is capable of selective reduction of an alkynylene group, an alkenylene group, an alkynyl group, an alkenyl group, a nitro group, an oxyalkylene group bonded to aromatic carbon, a carbobenzoxy group, an arylalkyloxy group bonded to aromatic carbon, an arylalkyloxy group bonded to carbonyl carbon, an aromatic carbonyl group, a halogeno group bonded to aromatic carbon, and an azide group out of the above-mentioned reduction target functional groups, and causes selective non-reduction of typically an arylalkyloxy group bonded to aliphatic carbon, an alkoxy group, an alkylcarbonyl group, and an oxyalkylene group bonded to aliphatic carbon out of the above-mentioned reduction non-target functional groups.

### C. Flow Reaction Apparatus

The supported palladium catalyst described in each of the above-mentioned sections A and B is applicable to any one of organic synthesis by a batch method (batch synthesis) and organic synthesis by a flow method (flow synthesis), and in particular, may be suitably adopted for the flow synthesis.

In one embodiment, a flow reaction apparatus capable of performing the flow synthesis includes the supported palladium catalyst. The supported palladium catalyst is typically loaded into a column in the flow reaction apparatus to form a fixed-bed catalyst.

When the supported palladium catalyst contains the fibrous carrier, the supported palladium catalyst is typically loaded into the column as it is.

In addition, when the supported palladium catalyst contains the particulate carrier, the flow reaction apparatus preferably further includes a filler to be mixed with the supported palladium catalyst. In this case, the supported palladium catalyst is loaded into the column in a state of being mixed into the filler, and is uniformly dispersed in the column.

A specific gravity of the filler with respect to the supported palladium catalyst (density of filler/density of supported palladium catalyst) is, for example, from 0.1 to 3.0, preferably from 0.5 to 2.0, more preferably 1.0±0.1.

When the specific gravity of the filler with respect to the supported palladium catalyst falls within the above-mentioned ranges, the supported palladium catalyst can be stably fixed in a fixed bed, and hence, in the flow synthesis, the supported palladium catalyst can be suppressed from flowing in the filler. Accordingly, the durability of the flow reaction apparatus can be improved.

In one embodiment, the supported palladium catalyst and the filler have forms identical to each other. An average primary particle diameter of the filler with respect to an average primary particle diameter of the supported palladium catalyst (average primary particle diameter of filler/average primary particle diameter of supported palladium catalyst) is, for example, from 0.1 to 3.0, preferably from 0.5 to 2.0, more preferably 1.0±0.1. The filler preferably includes a ceramics material having the same composition as that of the carrier in the supported palladium catalyst.

The mixing ratio of the filler is, for example, 300 parts by mass to 2,000 parts by mass, preferably 800 parts by mass to 1,200 parts by mass with respect to 100 parts by mass of the supported palladium catalyst.

In such flow reaction apparatus, a reactive substrate having at least the above-mentioned reduction target functional group can be continuously reduced. The reactive substrate preferably has a reduction non-target functional group in addition to the reduction target functional group. The reactive substrate is not particularly limited. Examples of the reactive substrate include compounds described in paragraph of [0038] to paragraph [0063] of JP 2015-180494 A.

In the continuous reduction reaction with the flow reaction apparatus, a reaction liquid obtained by dissolving the reactive substrate in any appropriate solvent is continuously supplied to the column. At this time, a hydrogen source (typically, hydrogen gas) is typically continuously supplied to the column together with the reaction liquid. Accordingly, when the reaction liquid passes through the column, hydrogen is donated from the hydrogen source to the reduction target functional group in the presence of the supported palladium catalyst to reduce the reduction target functional group. Thus, a desired target compound can be smoothly produced.

### Examples

The present disclosure is specifically described below by way of Examples, but the present disclosure is not limited by these Examples.

### <Palladium Catalyst>

### <<Example 1>>

Tetrakis(triphenylphosphine)palladium(0) serving as a palladium compound was heated under air at 80°C for 16 hours.

Next, 100 parts by mass of the heated palladium compound was dissolved in 100,000 parts by mass of chloroform serving as an organic solvent to prepare a palladium compound solution.

Next, under normal temperature and normal pressure (23°C, 0.1 MPa), a glass fiber was immersed in the palladium compound solution for 24 hours so that the palladium compound solution was brought into contact with a surface of the glass fiber. Thus, palladium(0) was supported on the surface of the glass fiber.

After that, the glass fiber was pulled up from the palladium compound solution, and then dried under reduced pressure at normal temperature (23°C) for 24 hours.

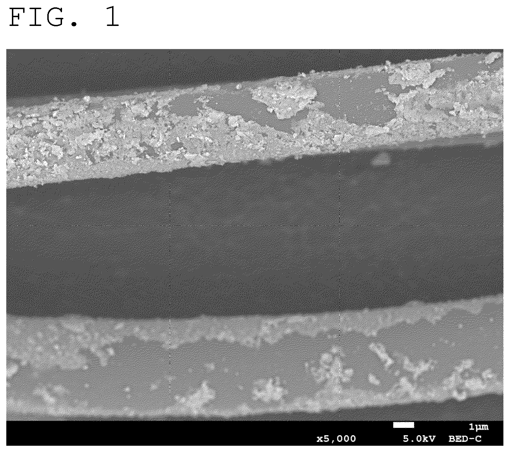

Accordingly, a palladium catalyst (Pd/GF) including the glass fiber serving as a carrier and palladium supported on the surface of the glass fiber was obtained. A backscattered electron image (BED) photograph of the palladium catalyst of Example 1 is shown in FIG. 1.

### <<Example 2>>

After a palladium compound solution was prepared in the same manner as in Example 1, silicon powder (Si carrier, particles classified into from 60 µm to 110 µm) was added to the palladium compound solution at a ratio of 100 parts by mass with respect to 3 parts by mass of palladium atoms, followed by replacement with an argon gas atmosphere, and the contents were stirred and mixed at normal temperature (23°C) for 24 hours. Thus, a dispersion liquid in which palladium(0) was supported on the silicon powder, and in which the silicon powder for supporting palladium(0) was dispersed in the organic solvent was prepared.

Next, the dispersion liquid was separated into a filtrate and a residue (solid content) by suction filtration. After that, the residue was washed with water, and then dried under reduced pressure at normal temperature (23°C) for 24 hours.

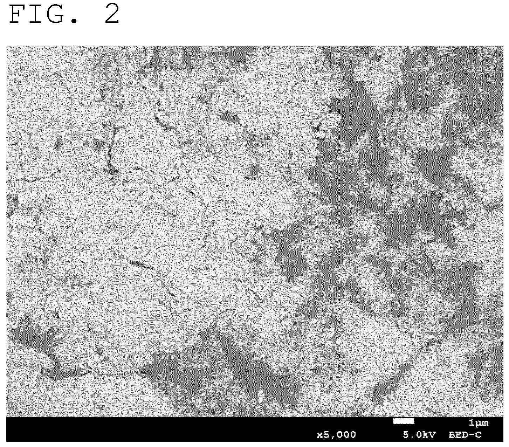

Thus, a powdery palladium catalyst (Pd/Si) was obtained. The palladium catalyst included the Si carrier and palladium supported on the surface of the Si carrier. A BED photograph of the palladium catalyst of Example 2 is shown in FIG. 2.

### <<Example 3>>

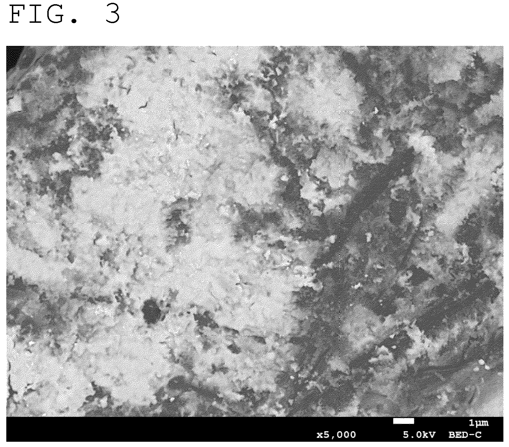

A powdery palladium catalyst (Pd/SiC) was obtained in the same manner as in Example 2 except that the silicon powder was changed to silicon carbide powder (SiC carrier, particles classified into from 60 µm to 110 µm). The palladium catalyst included the SiC carrier and palladium supported on the surface of the SiC carrier. A BED photograph of the palladium catalyst of Example 3 is shown in FIG. 3.

### <<Example 4>>

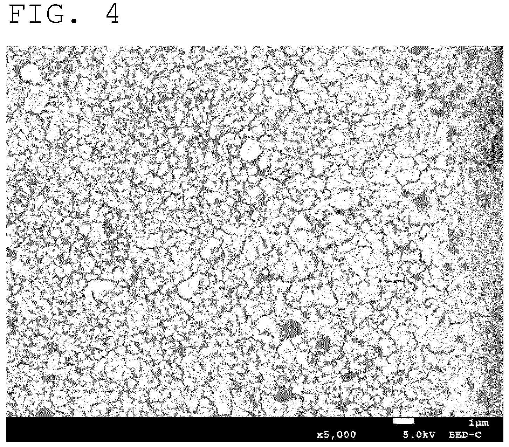

A powdery palladium catalyst (Pd/Si-SiC) was obtained in the same manner as in Example 2 except that the silicon powder was changed to Si-SiC-based composite material powder (Si-SiC carrier, particles classified into from 60 µm to 110 µm). The palladium catalyst included the Si-SiC carrier and palladium supported on the surface of the Si-SiC carrier. A BED photograph of the palladium catalyst of Example 4 is shown in FIG. 4.

### <<Example 5>>

A powdery palladium catalyst (Pd/C) was obtained in the same manner as in Example 2 except that: the silicon powder was changed to carbon particles (particulate activated carbon, C carrier, average primary particle diameter: 500 µm); and the carbon particles (particulate activated carbon) were added to the palladium compound solution at a ratio of 100 parts by mass with respect to 5 parts by mass of palladium atoms. The palladium catalyst included the C carrier and palladium supported on the surface of the C carrier.

### <Activity Evaluation>

9 Milligrams of each of the palladium catalysts of Examples 1 to 5, 1 mL of methanol, and 38 mg of 1-ethyl-4-nitrobenzene shown in Table 1 were loaded into a test tube, followed by replacement of the inside of the test tube with a hydrogen atmosphere. Next, an inner temperature of the test tube was adjusted to 25°C, and a reaction was performed for 24 hours.

After that, the reaction liquid in the test tube was recovered, and the solvent was removed from the reaction liquid. Thus, a solid content containing the reactive substrate and/or a reaction product was obtained. Next, the solid content was dissolved in deuterated chloroform, and the reactive substrate and the reaction product were analyzed with a nuclear magnetic resonance apparatus (¹H-NMR). The reactive substrate and the reaction product, and peak area ratios of the NMR spectra thereof are shown in Table 1.

**Table 1**

| No. | | | Example 1 | Example 2 | Example 3 | Example 4 | Example 5 |
|---|---|---|---|---|---|---|---|
| Catalyst configuration | | | Pd/GF | Pd/Si | Pd/SiC | Pd/ Si-SiC | Pd/C |
| Activity evaluation | Reactive substrate | 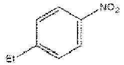 | 0 | 0 | 0 | 0 | 2 |
| | Reaction product | 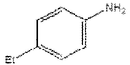 | 100 | 100 | 100 | 100 | 98 |

### <Evaluation>

As apparent from FIG. **1** to FIG. **4****,** it is found that, in the supported palladium catalysts of Examples 1 to 5, the palladium is sufficiently supported on surfaces of the various carriers. In each of the BED photographs of the supported palladium catalysts, it is observed that the palladium (relatively white part) is sufficiently supported on the surface of the carrier (relatively black part).

In addition, as shown in Table 1, it was recognized that those supported palladium catalysts were each capable of reducing a nitro group.

### Industrial Applicability

The supported palladium catalyst according to the embodiment of the present disclosure is appropriately used for various industrial products utilizing palladium as a catalyst, and can be suitably used as a reaction catalyst to be utilized in organic synthesis, in particular.

## Claims

1. A method of producing a supported palladium catalyst, comprising the steps of:
oxidizing a palladium compound by heating;
dissolving the palladium compound after the heating in a solvent to prepare a palladium compound solution; and
bringing the palladium compound solution into contact with a carrier.

2. The method of producing a supported palladium catalyst according to claim 1, wherein the palladium compound contains a palladium(0) complex containing zerovalent palladium.

3. The method of producing a supported palladium catalyst according to claim 2, wherein the palladium(0) complex contains tris(dibenzylideneacetone)dipalladium and/or tetrakis(triphenylphosphine)palladium.

4. A supported palladium catalyst, comprising:
a glass fiber serving as a carrier; and
palladium supported on a surface of the glass fiber.
